# EUROPÄISCHE PATENTSCHRIFT

(11) **EP 2 470 883 B1**
(45) Veröffentlichungstag und Bekanntmachung des Hinweises auf die Patenterteilung: **02.01.2019**
(21) Anmeldenummer: 10739581.6
(22) Anmeldetag: 28.07.2010
(51) Int. Cl.: G01N 21/85, G01N 21/11, B01L 3/00, G01N 35/10, F04B 43/08, F04B 43/12, G01N 21/03, G01N 21/05, G01N 21/78, G01N 21/84, G01N 33/18, G01N 1/40

(54) **PROZESS-ANALYSEGERÄT**
Process analyser
Appareil d'analyse de processus

(30) Priorität: 25.08.2009 EP 09168536; 01.04.2010 WO PCT/EP2010/054402
(43) Veröffentlichungstag der Anmeldung: 04.07.2012
(73) Patentinhaber: Hach Lange GmbH, 14163 Berlin (DE)
(72) Erfinder: LUNDGREEN, Ulrich, 33330 Gütersloh (DE); FARJAM, Aria, 40223 Düsseldorf (DE)
(74) Vertreter: Patentanwälte ter Smitten Eberlein-Van Hoof Rütten Partnerschaftsgesellschaft mbB
(86) Internationale Anmeldenummer: PCT/EP2010/060947
(87) Internationale Veröffentlichungsnummer: WO 2011/035959

(56) Entgegenhaltungen:
- EP-A- 1 870 033
- DE-A1- 10 126 054
- DE-A1- 10 214 781
- DE-A1- 10 227 032
- US-A- 4 583 920
- US-A- 5 593 290
- US-A- 5 646 863
- US-A1- 2004 013 536
- US-A1- 2008 026 373
- US-B1- 6 852 284
- DANFOSS: "EVITA - In situ sensors for measuring ammonium, nitrate or orthophosphate" DANFOSS DATA SHEET, XX, XX, 1. August 1997 (1997-08-01), Seite 4PAGES, XP002989216
- WANG C H ET AL: "Automatic bio-sampling chips integrated with micro-pumps and micro-valves for disease detection", BIOSENSORS AND BIOELECTRONICS, ELSEVIER BV, NL, vol. 21, no. 3, 15 September 2005 (2005-09-15), pages 419-425, XP027619412, ISSN: 0956-5663 [retrieved on 2005-09-15]

## Beschreibung

Die Erfindung bezieht sich auf ein Prozess-Analysegerät, wie es beispielsweise in der Wasser-Analytik in Form einer Tauchsonde, einer Rohreinbausonde oder eines Labor-Analysegerätes eingesetzt wird.

Prozess-Analysegeräte führen quasi-kontinuierlich Analysen zur quantitativen Bestimmung eines Analyts in Wasser durch, und weisen hierfür einen oder mehrere Vorratstanks zur Bevorratung von Flüssigkeiten, z.B. Trägerflüssigkeiten, Standardlösungen und/oder Reagenzien auf, die für die Durchführung der Wasser-Analyse benötigt werden. Für den Transport der Flüssigkeiten in dem Analysegerät sind Pumpen und Flüssigkeitsleitungen vorgesehen.

Bei Erschöpfung eines Flüssigkeits-Vorrats in einem Vorratstank muss der betreffende Vorratstank wieder aufgefüllt werden. Im praktischen Betrieb treten ferner häufig Störungen auf, die insbesondere durch Ablagerungen und Auskristallisationen in den Flüssigkeitsleitungen oder auch in den flüssigkeitsführenden Teilen der Pumpen verursacht werden. Diese Störungen, sowie normale Abnutzung von Verschleißteilen wie z.B. Schlauchpumpen-Schläuchen, führen dazu, dass zusätzlich zu der regelmäßigen Auffüllung bzw. Austausch der Vorratstanks auch Wartungsarbeiten und Reparaturarbeiten notwendig sind, z.B. Reinigung und Wartung der Pumpen, Austausch von Schläuchen etc.

Aus DE 101 26054 A1 ist ein Analysegerät bekannt, das aus einem Basismodul und einem austauschbaren Kartuschenmodul besteht. Das Basismodul weist eine Analyseeinheit auf, so dass zwischen dem Basismodul und dem Kartuschenmodul eine Vielzahl von Flüssigkeitsleitungs-Schnittstellen vorhanden ist. Alternativ kann die die Analyseeinheit auch vollständig in dem Kartuschenmodul angeordnet sein, so dass in diesem Fall eine Vielzahl elektrischer Schnittstellen zwischen dem Basismodul und dem Kartuschenmodul erforderlich ist.

Aufgabe der Erfindung ist es demgegenüber, ein Prozess-Analysegerät mit hoher Betriebssicherheit und geringem Wartungsaufwand zu schaffen.

Diese Aufgabe wird erfindungsgemäß durch ein Prozess-Analysegerät mit den Merkmalen des Patentanspruches 1 gelöst.

Das erfindungsgemäße Prozess-Analysegerät ist modular aufgebaut, und besteht aus einem ständig verwendeten Basismodul und einem austauschbaren Kartuschenmodul.

Das Basismodul weist einen Pumpen-Antriebsmotor und einen Analyt-Sensor ohne flüssigkeitsführende Messtrecke auf. Das Basismodul weist keinerlei Fluidik auf, also keine flüssigkeitsführenden Leitungen, Ventile, Messstrecken oder Pumpmimiken.

Das Kartuschenmodul dagegen weist mindestens einen Flüssigkeits-Vorratstank, eine antriebslose Pumpmimik zum Pumpen der Flüssigkeit aus dem Vorratstank, und eine Messstrecke für den Analyt-Sensor auf. Die gesamte Fluidik ist also In dem Kartuschenmodul angeordnet. Unter Pumpmimik ist der fluidische Transportmechanismus zu verstehen, d.h. der Teil der Pumpe, der in direktem Kontakt zur Flüssigkeit steht und den Transport innerhalb des fluidischen Systems bewirkt.

Bei in das Basismodul eingesetztem Kartuschenmodul wird die Pumpmimik über das Kupplungsteil durch den Pumpenantrieb angetrieben, und ist die flüssigkeitsführende Messstrecke dem Analyt-Sensor funktional derart zugeordnet, dass dieser Messungen von in der Flüssigkeit enthaltenen Analyten durchführen kann.

Bei Erschöpfung der Flüssigkeit in dem Vorratstank, beispielsweise einer Erschöpfung von Trägerflüssigkeit oder Reagenz, wird das Kartuschenmodul mit der gesamten Fluidik ausgetauscht. Der Austausch der gesamten Fluidik erfolgt also regelmäßig beziehungsweise verbrauchsgesteuert. Eine separate Wartung bzw. verschleißbedingte Reparatur der Fluidik des Analysegerätes entfällt. Der Wartungsaufwand ist dadurch erheblich reduziert. Durch das regelmäßige beziehungsweise verbrauchsgesteuerte Austauschen der gesamten Fluidik werden Störungen, die durch Ablagerungen in der Fluidik oder Verschleiß fluidischer Komponenten, z.B. Pumpenschläuche, verursacht werden können, auf ein Minimum reduziert.

Erfindungsgemäß ist die Pumpmimik ausgebildet als pneumatische peristaltische Membranpumpe. Eine peristaltische Pumpmimik ist konstruktiv sehr einfach, und kann beispielsweise von einem flexiblen Abschnitt der Flüssigkeitsleitung gebildet werden, der von dem Kopplungsteil in einer Längsrichtung fortlaufend verformt wird.

Das Kopplungsteil kann beispielsweise in Form von drei in Längsrichtung hintereinander angeordneten Quetschkolben ausgebildet sein, die in einer Längsrichtung nacheinander den Flüssigkeitsleitungs-Abschnitt flüssigkeitsdicht abquetschen, und auf diese Weise eine peristaltische Pumpbewegung erzeugen. Eine besonders bevorzugte Ausführungsform ist die pneumatische Peristaltikpumpe, bei der die mechanische Kopplung durch Anlegen von Über- bzw. Unterdruck auf eine mehrstufige Membranpumpe erzeugt wird. Der Pumpenantrieb ist pneumatisch ausgebildet. Ein Lufteintrag in einer Flüssigkeitsleitung stellt für eine peristaltische Pumpmimik kein Problem dar, da diese gegen Luft ansaugen kann. Ferner kann bei Verwendung einer peristaltischen Pumpmimik auf Ventile, insbesondere auf anfällige Rückschlag-Ventile verzichtet werden. Die peristaltische Pumpmimik bietet ferner die Möglichkeit, mit einer einzigen peristaltischen Pumpmimik mehrere Leitungen synchron pumpen zu können, also mehrere Flüssigkeiten gleichzeitig. Die peristaltische Pumpmimik ist eine ausgereifte, preiswerte und äußerst zuverlässige Pumptechnik.

Erfindungsgemäß weist das Basismodul mehrere voneinander unabhängige Pumpenantriebe auf, die mehreren voneinander unabhängigen Pumpmimiken in dem Kartuschenmodul zugeordnet sind.

Zwar können grundsätzlich auch mit einem einzigen Pumpenantrieb mehrere Pumpmimiken angetrieben werden. Vorteilhaft ist jedoch, jeder Pumpmimik in dem Kartuschenmodul basismodulseitig einen eigenen Pumpenantrieb zuzuordnen. Auf diese Weise können mehrere Flüssigkeitsströme unabhängig voneinander gesteuert werden.

Vorzugsweise weist das Kartuschenmodul mehrere Vorratstanks auf. Als Vorratstank ist vorliegend ein Tank für ein Reagenz, eine Trägerflüssigkeit, eine Standardlösung, eine Reinigungsflüssigkeit oder auch für Abfallflüssigkeit zu verstehen. Insbesondere bei einem Analysegerät mit dialytischer Probengewinnung kann auf diese Weise sichergestellt werden, dass zwischen zwei Kartuschenmodul-Wechseln keinerlei Flüssigkeit aus dem Analysegerät austritt.

Gemäß einer bevorzugten Ausgestaltung weist der Analyt-Sensor basismodulseitig ein Fotometer mit einem optischen Detektor zur fotometrischen Bestimmung eines Analyts in einer Probe auf. Der optische Detektor beziehungsweise das Fotometer bestimmt die Absorption, die Fluoreszenz oder die Lumineszenz des Analyts in der Probe, die sich in der Fluidik-Messstrecke befindet.

Die Probe kann beispielsweise dialytisch aus dem das Analysegerät umgebenden Wasser, beispielsweise Abwasser, gewonnen werden. Hierzu weist das Kartuschenmodul in einer bevorzugten Ausführungsform eine Dialysemembran auf. Bei der dialytischen Probengewinnung gelangt das zu untersuchende Wasser nicht in das Analysegerät, sondern wandern lediglich Moleküle und Ionen aus dem Wasser in eine Trägerflüssigkeit jenseits der Dialysemembran. Das Flüssigkeitsvolumen innerhalb des Analysegerätes verändert sich also während der gesamten Betriebszeit zwischen zwei Kartuschenmodul-Wechseln nicht wesentlich. Die Abfallflüssigkeit kann also in dem Analysegerät gesammelt werden, und gelang nicht in das umgebende Wasser.

Besonders bevorzugt ist das vorliegende Analysegerät daher als flüssigkeitsdichte Abwasser-Tauchsonde ausgebildet, die direkt in das zu untersuchende Wasser, beispielsweise Abwasser in einem Becken einer Kläranlage, eingetaucht betrieben wird.

Erfindungsgemäß weist das Kartuschenmodul eine Kunststoff-Platte auf, die die Pumpkammern der Pumpmimik und nutartige fluidische Kanäle mit einem Kanal-Querschnitt von weniger als 5 mm² zum Verbinden des Vorratstanks, der Pumpmimik und der Messstrecke aufweist. Auf der Kunststoff-Platte ist im Bereich der Pumpkammern eine flexible und flüssigkeitsdichte Pumpenmembran und vorzugsweise im Bereich der Kanäle eine steife Abdeckung vorgesehen, die die Kanäle verschließt. Vorzugsweise ist die gesamte Fluidik mikrofluidisch ausgebildet, so dass die Kanäle bevorzugt einen Querschnitt von höchstens 10,0 mm², bevorzugt von höchstens 5,0 mm² aufweisen. Nur mit einer mikrofluidischen Fluidik ist es möglich, ein Analysegerät zu schaffen, das über einen wirtschaftlich ausreichend langen Zeitraum mit den Flüssigkeiten in den Vorratstanks auskommt.

Im Folgenden wird die Erfindung anhand der Zeichnungen näher erläutert. Es zeigen:
- Figur 1: eine schematische Darstellung eines als Tauchsonde ausgebildeten ersten Ausführungsbeispiels eines Analysegerätes,
- Figur 2: eine erste Ausführungsform eines basisseitigen pneumatischen Pumpenantriebs und einer damit zusammenwirkenden kartuschenseitigen Pumpmimik, und
- Figur 3: eine Beispiel eines basisseitigen elektromechanischen Pumpenantriebs und einer damit zusammenwirkenden kartuschenseitigen Pumpmimik.
- Figur 4: eine schematische Darstellung eines zweiten Ausführungsbeispiels eines Prozess-Analysegerätes,
- Figur 5: einen Längsschnitt durch eine weitere Ausführungsform eines Analysegerätes, und
- Figur 6: eine Draufsicht auf das Kartuschenmodul des Analysegerätes der Figur 5.

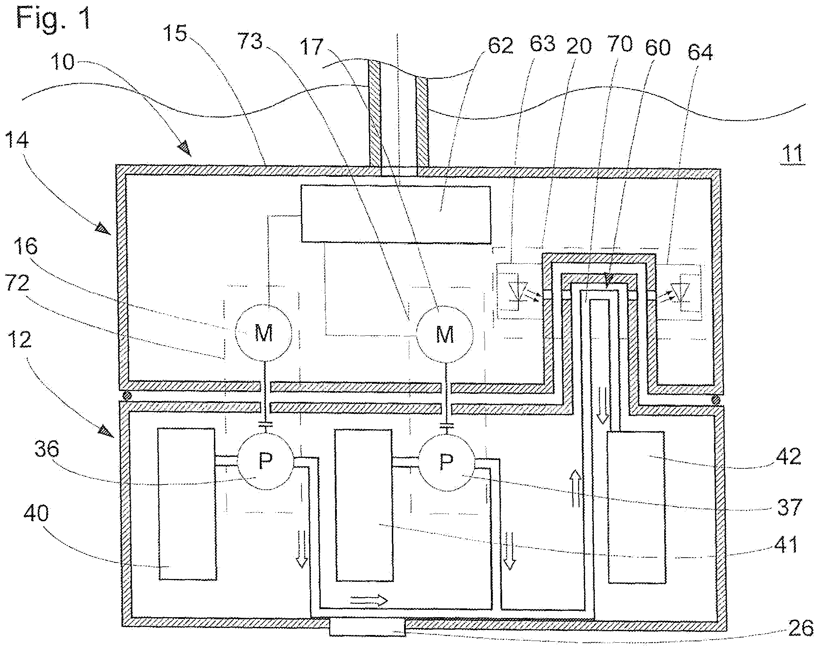

In der Figur 1 ist schematisch ein Prozess-Analysegerät 10 dargestellt, das als Tauchsonde ausgebildet Ist, und der Analyse von Wasser 11 zur Regelung eines Klärprozesses dient. Das Analysegerät 10 ist zweiteilig und modular aufgebaut, und besteht aus einem flüssigkeitsdichten Basismodul 14 und einem austauschbaren flüssigkeitsdichten Kartuschenmodul 12. Das Basismodul 14 weist ein Gehäuse 15 auf, in das bzw. an das das Kartuschenmodul 12 ein- bzw. angesteckt ist.

Das Basismodul 14 weist einen als Fotometer ausgebildeten Analyt-Sensor 20 ohne Fluidik-Messstrecke 60 auf, die in dem Kartuschenmodul 12 vorgesehen ist. Der Analyt-Sensor 20 weist basismodulseitig eine Lichtquelle 63 und einen optischen Detektor 64 in Form einer Photozelle auf.

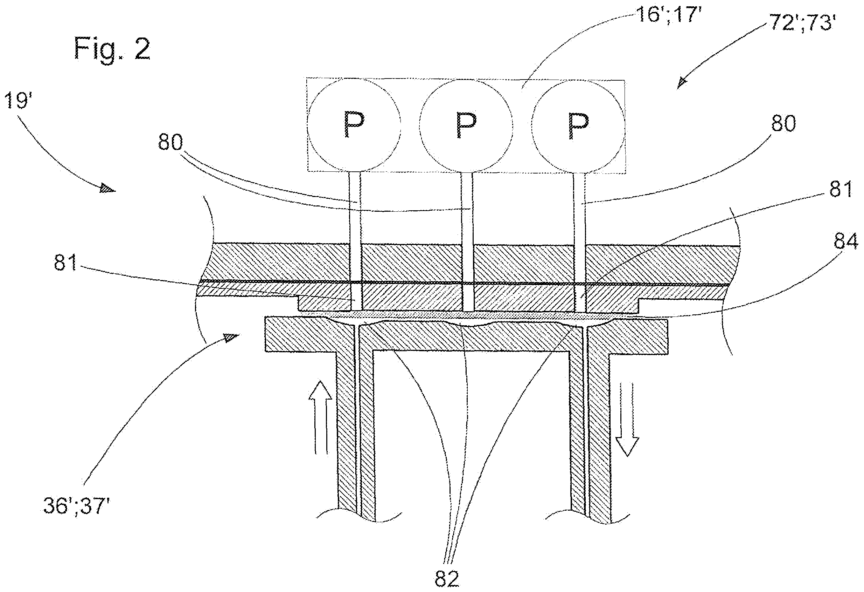

Wie in Figur 2 dargestellt ist, kann die Pumpe gemäß einer ersten Ausführungsform pneumatisch ausgebildet sein, wobei der Pumpenantrieb 16' als Luftpumpe ausgebildet ist, die drei Luftkanäle 80 sukzessive mit Überdruck und ggfs. auch mit Unterdruck beaufschlagt, die mit entsprechenden Luftkanälen 81 des Kartuschenmoduls 12 gekoppelt sind. Die kartuschenmodulseitigen Luftkanäle 81 sind durch eine Pumpmembran 84 verschlossen, unter der drei Pumpkammern 82 gebildet sind.

Wird in dem Luftkanal 80 durch den Pumpenantrieb 16' Überdruck angelegt, dehnt sich die Pumpmembran 84 in die darunter liegende Pumpkammer 82 aus und verdrängt die darin befindliche Flüssigkeit. Wird Unterdruck angelegt, legt sich die Pumpmembran 84 glatt an die Oberfläche der Luftseite an und die Pumpkammer 82 füllt sich wieder mit Flüssigkeit. Durch wechselndes Anlegen von Über- und Unterdruck in der entsprechenden Reihenfolge der drei aufeinander folgenden Pumpkammern 82 wird eine peristaltische Fortbewegung der Flüssigkeit durch alle drei Pumpkammern 82 und im nachfolgenden fluidischen System erreicht.

In dem Pumpenantrieb 16' sind drei nicht dargestellte Ventile vorgesehen, die entweder Überdruck oder Unterdruck in den jeweiligen Luftkanal 80 durchschalten. Die in den Figuren nicht dargestellten Ventile sind demnach die Kopplung zwischen der Druckluftpumpe und der Pumpmimik 36'.

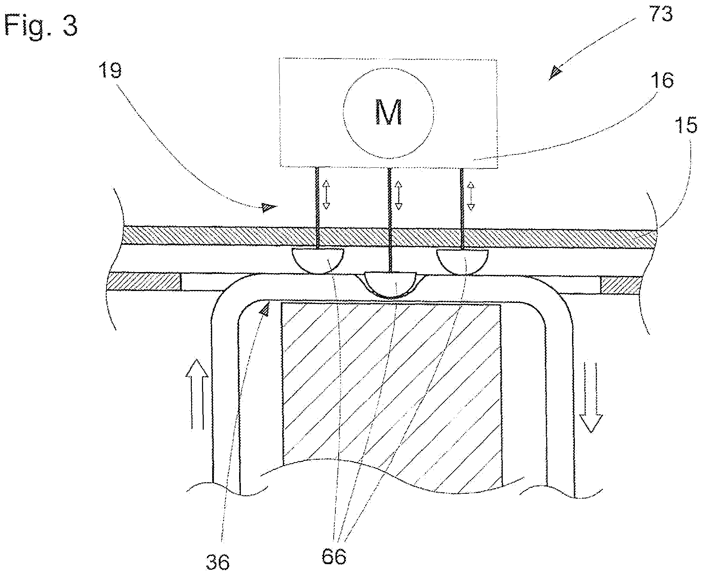

Das Basismodul 14 weist als Pumpenantrieb in einem Beispiel einen Antriebsmotor 16,17 mit einem Kopplungsteil 19 auf, das in der Figur 3 dargestellt ist. Das Kopplungsteil 19 besteht aus drei peristaltischen Quetschkolben 66, die eine peristaltische Bewegung in einer als flexibler Leitungsabschnitt ausgebildeten Pumpmimik 36 erzeugen können.

Zur Steuerung des Analysegerätes 10 weist das Basismodul 14 ferner eine Steuerung 62 auf.

Das Kartuschenmodul 12 weist drei Vorratstanks 40,41,42 auf, in denen eine Trägerflüssigkeit, ein Reagenz sowie Abfallflüssigkeit gelagert sind. Der Trägerflüssigkeits-Tank 40 und der Reagenz-Tank 41 sind jeweils über eine Flüssigkeitsleitung an eine eigene peristaltische Pumpmimik 36,37 angeschlossen, durch die die Trägerflüssigkeit beziehungsweise das Reagenz fein dosiert gepumpt werden kann. Der dritte Vorratstank 42 ist ein Tank für Abfallflüssigkeit, in den die von dem Analysator 20 kommende Abfall-Flüssigkeit fließt.

Die peristaltischen Pumpmimiken 36,37 des in der Figur 3 dargestellten Beispiels sind zusammen mit den zugeordneten Kopplungsteilen 19 derart ausgelegt, dass ein unerwünschter Rückfluss oder Leckfluss von Trägerflüssigkeit oder Reagenz nicht auftreten kann. Die peristaltischen Pumpmimiken 36,37 sind kartuschenmodulseitig im Wesentlichen durch flexible Abschnitte der betreffenden Flüssigkeitsleitung gebildet, die durch die betreffenden Kopplungsteile 19 der zugeordneten Antriebsmotoren 16,17 angetrieben werden. Die Fluidik- Pumpe 72,73 der zweiten Ausführungsform wird also von dem Antriebsmotor 16, dem Kopplungsteil 19 und der Pumpmimik 36 gebildet.

Ferner weist das Kartuschenmodul 12 eine ionenselektive Dialysemembran 26 auf, durch die Analyt-Moleküle und -Ionen aus dem Wasser 11 in die innenseitig vorbei fließende Trägerflüssigkeit wandern können, Partikel, Makromoleküle und Mikroorganismen aber vom Membran-Durchtritt abgehalten werden. Die auf diese Weise mit denen Analyt-Moleküle und - Ionen angereicherte Trägerflüssigkeit bildet die so genannte Probe. Die Dialysemembran 26 weist bevorzugt eine Porengröße von weniger als 2 µm auf.

Schließlich weist das Kartuschenmodul 12 die Fluidik-Messstrecke 60 auf, die von einem geraden Leitungsabschnitt 70 gebildet wird, dessen Längsenden transparente, lichtdurchlässige Fenster für die Fotometrie durch den Analyt-Sensor 20 aufweisen.

Wenn das Kartuschenmodul 12 an dem Basismodul 14 montiert ist, ist die Messtrecke 60 genau ausgerichtet mit den basismodulseitigen Teilen des Analyt-Sensors 20. Ferner sind die Kopplungsteile 19 der Pumpen 72, 73 in peristaltischem Eingriff mit den kartuschenmodulseitigen Pumpmimiken 36.

Im Analysebetrieb wird aus dem Trägerflüssigkeits-Vorratstank 40 durch die zugeordnete Pumpe 72 Trägerflüssigkeit zu der Dialysemembran 26 gepumpt, durch die Analyt -Ionen beziehungsweise -Moleküle aus dem Wasser 11 in die Trägerflüssigkeit wandern. Anschließend wird der mit den Analyt -Ionen angereicherten Trägerflüssigkeit, der so genannten Probe, ein Reagenz aus dem Reagenz-Vorratstank 41 durch die zugeordnete Pumpe 73 beigefügt, durch das das zu untersuchende Analyt in der Trägerflüssigkeit entsprechend seiner Konzentration eingefärbt wird. Die eingefärbte Probe wird zu der Messstrecke 60 gepumpt und dort durch den Analyt-Sensor 20 fotometrisch untersucht, wobei die Konzentration des Analyts in der so genannten Probe bestimmt werden kann, die wiederum einen Rückschluss auf die Konzentration des Analyts in dem das Analysegerät umgebenden Wasser erlaubt. Anschließend wird die Probe in den Abfall-Vorratstank 42 gepumpt.

Bei Erschöpfung von Reagenz oder Trägerflüssigkeit in den entsprechenden Vorratstanks 40,41, bei drohender Überfüllung des Abfall-Vorratstanks 42 oder bei einer Fehlfunktion, die möglicherweise auf eine der Komponenten des Kartuschenmoduls 12 zurückgeht, wird das Kartuschenmodul 12 ausgetauscht. Hierbei wird eine nicht dargestellte Verriegelung entriegelt und das Kartuschenmodul 12 axial nach unten von dem Basismodul-Gehäuse 15 abgezogen. Hierbei wird das Pumpen-Kopplungsteil 19 von der Pumpmimik 36 gelöst und wird die Messtrecke 60 aus dem Analyt-Sensor 20 entfernt. Anschließend kann ein neues Kartuschenmodul 12 an das Gehäuse 15 angesetzt werden, wobei die Pumpen-Kopplungsteile 19 wieder mit den Pumpmimiken 36 verbunden werden und die Messstrecke 60 in den Analysator 20 eingeführt wird.

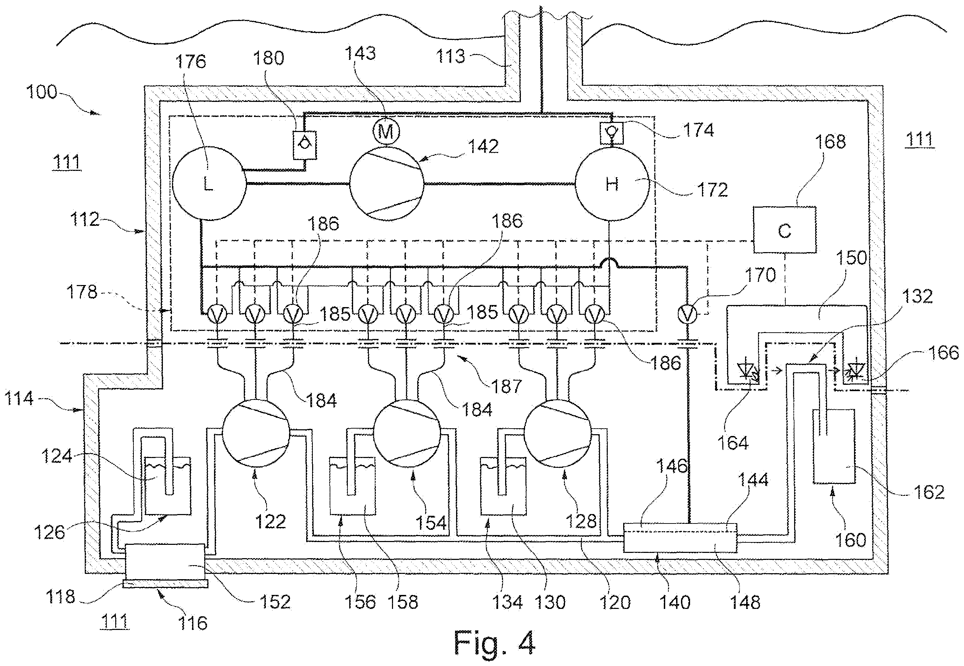

In der Figur 4 ist schematisch ein zweites Ausführungsbeispiel eines Prozess-Analysegerätes 110 zur kontinuierlichen beziehungsweise quasikontinuierlichen quantitativen fotometrischen Bestimmung eines Analyten, beispielsweise Phosphat, Ammonium oder Nitrat, in Wasser dargestellt. Das Analysegerät 110 ist ein stationäres Analysegerät 110 und ist in das zu untersuchende Wasser 111 eingetaucht montiert, ist also als so genannte Tauchsonde ausgebildet. Das Analysegerät 110 besteht aus einem Basismodul 112, das an einem Rohrgestänge 113 starr in dem Wasser 111, also der Analyseflüssigkeit, oder knapp darüber aufgehängt ist, und einem austauschbaren Kartuschenmodul 114, das an dem Basismodul 112 lösbar befestigt und in das Wasser 111 eingetaucht ist. Die gesamte Fluidik des Analysegerätes 110 ist in dem Kartuschenmodul 114 vorgesehen. Das Kartuschenmodul 114 weist einen Trägerflüssigkeits-Vorratstank 126 mit einer Trägerflüssigkeit 124 auf, die über eine Leitung an eine Probengewinnungsvorrichtung 116 angeschlossen ist, die vorliegend eine Dialysevorrichtung 116 ist. Die Dialysevorrichtung 116 weist als Membran 118 eine Dialysemembran 118 auf, die den Dialyseraum 152, in dem die Trägerflüssigkeit während der Dialyse verweilt, von dem Wasser 111 trennt. Der Dialyseraum 152 kann beispielsweise von einer mäanderartig verlaufenden Nut gebildet werden, deren Nutöffnung durch die Dialysemembran 118 verschlossen ist. Hinter der Dialysevorrichtung 116 ist eine erste Pumpmimik 122 vorgesehen, die die Probe 120 bzw. das Dialysat aus der Dialysevorrichtung 116 zu einer Entgasungsvorrichtung 140 pumpt.

Das Kartuschenmodul 114 weist einen Reagenz-Vorratstank 134 mit einem flüssigen Reagenz 130 auf, das durch eine zweite Pumpmimik 128 zu der Entgasungsvorrichtung 140 gepumpt wird. Ferner ist in dem Kartuschenmodul 114 ein Standardlösungs-Vorratstank 156 mit einer Standardlösung 158 vorgesehen, wobei in Fließrichtung gesehen hinter dem Standardlösungs-Vorratstank 156 eine dritte Pumpmimik 154 vorgesehen ist, die die Standardlösung bei Bedarf zu der Entgasungsvorrichtung 140 pumpt.

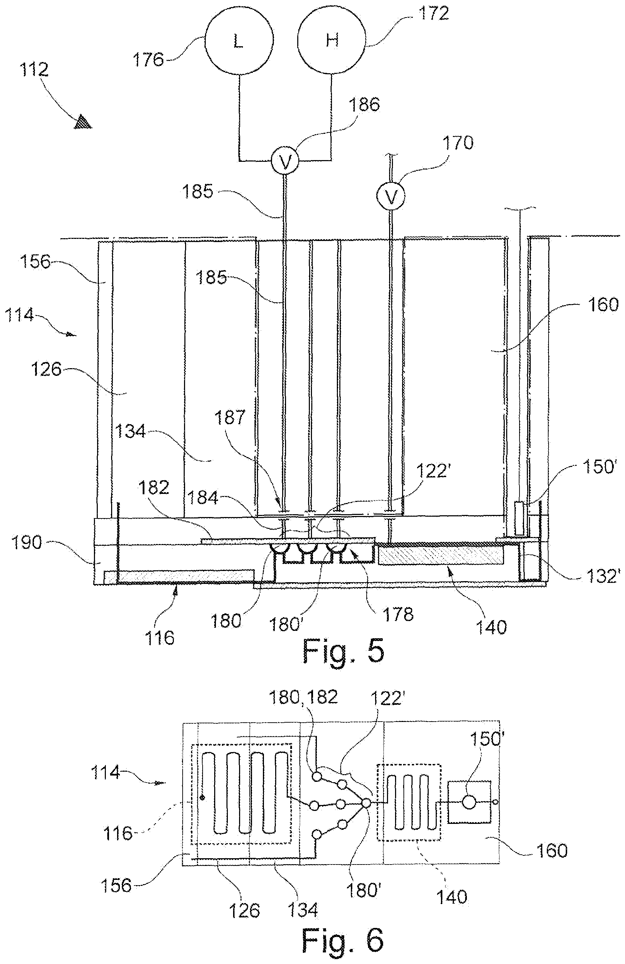

Die drei Pumpmimiken 122, 128, 154 laufen kurz vor der Entgasungsvorrichtung 140 sternförmig zusammen, wie insbesondere in Figur 6 gut zu erkennen ist. Die Entgasungsvorrichtung 140 wird von einem nutförmigen Entgasungskanal 148 gebildet, der durch eine gasdurchlässige und flüssigkeitsdichte Entgasungsmembran 144 bedeckt ist, die eine hydrophobe Teflon™-Membran ist. Der Entgasungskanal 148 verläuft mäanderartig, so dass auf einer kleinen Fläche ein relativ langer Entgasungskanal 148 realisiert ist. Auf der dem Entgasungskanal 148 gegenüberliegenden Seite der Entgasungsmembran 144 ist die Entgasungsvorrichtungs-Gasseite 146 angeordnet, deren Evakuierung über ein basismodulseitiges Entgasungs-Ventil 170 gesteuert wird.

Die Probe fließt von der Entgasungsvorrichtung 140 zu einer Messstrecke 132, und von dort aus in einen Abfallflüssigkeits-Tank 160, in dem die Abfallflüssigkeit 162 gesammelt wird. Die Messstrecke 132 ist funktional einem basismodulseitigen fotometrischen Analyt-Sensor 150 zugeordnet, der eine Lichtquelle 164 und einen optischen Detektor 166 aufweist, zwischen denen ein die Messstrecke bildender Abschnitt der Dialysat-Leitung in Längsrichtung angeordnet ist. Der Analyt-Sensor 150 ist vorliegend als Transmissions-Fotometer ausgebildet. Der Analyt-Sensor 150' kann alternativ jedoch auch als Reflektions-Fotometer ausgebildet sein, wie in dem Ausführungsbeispiel der Figur 5.

Als Druckquellen zum Antrieb der drei Pumpmimiken 122,154,128 dienen ein Überdruck-Speicher 172 und ein Unterdruck-Speicher 176 in dem Basismodul 112. Den drei Pumpmimiken 122,154,128 sind als pneumatische Peristaltikpumpen ausgebildet. Jeder Pumpmimik 122,154,128 ist jeweils eine Ventil-Bank zugeordnet, die jeweils aus drei Umschaltventilen 186 besteht. Jede Pumpmimik 121,154,128 weist jeweils drei Pumpkammern 180 mit jeweils einer elastischen Pumpenmembran 182 auf, die aus Gummi oder aus einem elastischen Kunststoff besteht.

Die Rückseite der Pumpenmembran 182 ist jeweils über eine kartuschenmodulseitige pneumatische Steuerleitung 184, eine Steuerleitungs-Kupplung 187 und eine basismodulseitige pneumatische Steuerleitung 185 mit einem Umschaltventil 186 verbunden, das die Pumpenmembran 182 wahlweise mit dem Überdruck-Speicher 172 oder dem Unterdruck-Speicher 176 verbindet. Auf diese Weise wird die Rückseite der Pumpenmembran 182 entweder mit Überdruck oder mit Unterdruck beaufschlagt, so dass die Pumpkammern 180 gefüllt oder geleert werden. Durch sukzessives Füllen und Leeren der drei Pumpkammern 122,154,128 wird eine peristaltische Pumpbewegung generiert.

Zur Erzeugung des Unterdrucks in dem Unterdruck-Speicher 76 und des Überdrucks in dem Überdruck-Speicher 172 ist eine Pneumatikpumpe 142 in dem Basismodul 112 vorgesehen, deren Pumpeneinlass mit dem Unterdruck-Speicher 176 und deren Pumpenauslass mit dem Überdruck-Speicher 172 verbunden ist. Die Pneumatikpumpe 142 wird ständig durch einen elektrischen Pneumatikpumpen-Motor 143 angetrieben. Der Unterdruck in dem Unterdruck-Speicher 176 und der Überdruck in dem Überdruck-Speicher 172 wird jeweils durch ein entsprechendes Unterdruckventil 180 beziehungsweise Überdruckventil 174 begrenzt, die jeweils mit atmosphärischem Luftdruck verbunden sind. Alternativ können in den Speichern 172, 176 auch Drucksensoren vorgesehen sein, durch die bei Über- bzw. Unterschreiten eines Grenzdruckes die Pneumatikpumpe ein- bzw. ausgeschaltet wird.

Die gesamte Pneumatik, die im Wesentlichen von den Ventilen 185, den Druckspeichern 172, 176, der Pneumatikpumpe 142 und dem Motor 143 gebildet wird, bildet den jeweiligen unabhängigen Pumpenantrieb 178 für die Pumpmimiken 122, 154, 128.

Das den Unterdruck in der Entgasungsvorrichtung 140 steuernde Entgasungs-Ventil 170 ist an den Unterdruck-Speicher 176 angeschlossen.

Alle Ventile 186,170 und der Analyt-Sensor 150 werden durch eine zentrale Steuerung 168 gesteuert. Alle elektrischen Bauteile sind in dem Basismodul 112 angeordnet.

In den Figuren 5 und 6 ist eine weitere Ausführungsform eines Analysegerätes beziehungsweise eines Kartuschenmoduls 114 dargestellt. Ein Unterschied zu der in der Figur 4 lediglich schematisch dargestellten Ausführungsform ist die konkrete Ausgestaltung der drei Pumpmimiken 122', deren jeweils letzte Pumpkammer 180' von einer einzigen gemeinsamen Pumpkammer 180' gebildet wird. Ein weiterer Unterschied bei der Ausführungsform der Figur 5 Ist die Ausbildung des Analyt-Sensors 150' als Reflektions-Fotometer.

Wie in den Figuren 5 und 6 gut erkennbar ist, besteht das Kartuschenmodul 114 im wesentlichen aus einem plattenförmigen Kunststoff-Teil 190, das die Fluidikleitungen, die Pumpkammern 180,180', das Dialysemodul 116, die Entgasungsvorrichtung 140 und die Messstrecke 132' aufweist, und aus den Tanks 126, 134,156, 162, die auf das plattenförmige Kunststoff-Teil 190 aufgesetzt sind.

## Patentansprüche

1. Prozess-Analysegerät (110) zur Bestimmung eines Analyts in Wasser, mit einem Basismodul (112) und einem austauschbaren Kartuschenmodul (114), wobei
das Basismodul (112) keine flüssige Fluidik aufweist, wobei das Basismodul (112) einen Pumpenantrieb (16',17';178) und einen Analyt- Sensor (150) ohne Fluidik-Messstrecke (132) aufweist, und das Kartuschenmodul (114) die gesamte flüssige Fluidik aufweist, wobei das Kartuschenmodul (114) einen Flüssigkeits-Vorratstank (126,156,134), eine Probengewinnungs-Vorrichtung, eine antriebslose Pumpmimik (36';122,154,128), die die Flüssigkeit aus dem Vorratstank (126,156,134) pumpt, und die Fluidik-Messstrecke (132) für den Analyt-Sensor (150) aufweist, wobei
bei in das Basismodul (112) eingesetztem Kartuschenmodul (114) die Pumpmimik (36';122,154,128) mit dem Pumpenantrieb (16';178) verbunden und durch den Pumpenantrieb (16';178) angetrieben wird, und die Fluidik-Messstrecke (132) mit dem Analyt-Sensor (150) verbunden ist,
die Pumpmimik (36'; 122,154,128) als peristaltische Membranpumpe ausgebildet und pneumatisch angetrieben ist,
die Pumpkammern (82) der Membranpumpe durch eine flexible flüssigkeitsdichte Pumpenmembran (84) bedeckt sind,
das Basismodul (112) mehrere voneinander unabhängige Pumpenantriebe (16",17";178) aufweist, die mehreren voneinander unabhängigen Pumpmimiken (36',37'; 122,154,128) in dem Kartuschenmodul (114) zugeordnet sind, und
das Kartuschenmodul (114) eine Kunststoff-Platte (190) aufweist, die nutartige mikrofluidische Kanäle mit einem Kanal-Querschnitt von weniger als 5 mm² zum Verbinden des Vorratstanks (126,156,134), der Pumpmimiken (123,154,128) und der Messstrecke (132) aufweist.

2. Prozess-Analysegerät (10:110) nach Anspruch 1, wobei das Kartuschenmodul (12;114) mehrere Vorratstanks (40,41;126,156,134) aufweist.

3. Prozess-Analysegerät (10:110) nach einem der vorangegangenen Ansprüche, wobei der Analyt-Sensor (20;150) einen optischen Detektor (64;166) aufweist, der die optische Bestimmung eines Analyts in der Fluidik-Messstrecke (60;132) erlaubt.

4. Prozess-Analysegerät (10;110) nach Anspruch 3, wobei der optische Detektor (64;166) die Absorption, die Fluoreszenz oder die Lumineszenz der Probe ermittelt.

5. Prozess-Analysegerät (10:110) nach einem der vorangegangenen Ansprüche, wobei das Kartuschenmodul (14;114) eine DialyseMembran (26;118) zur Gewinnung einer Probe aufweist.

6. Prozess-Analysegerät (10:110) nach einem der vorangegangenen Ansprüche, wobei das Analysegerät (110) als Tauchsonde ausgebildet ist.

7. Prozess-Analysegerät (10;110) nach einem der vorangegangenen Ansprüche, wobei das Kartuschenmodul (114) flüssigkeitsdicht gekapselt ist, so dass die darin enthaltenen Flüssigkeiten nicht nach außen treten können.

## Claims

1. A process analysis unit (110) for the determination of an analyte in water, comprising a base module (112) and an exchangeable cartridge module (114), wherein
the base module (112) comprises no liquid fluidic system, the base module (112) comprising a pump drive (16', 17'; 178) and an analyte sensor (150) without a fluidic measuring section (132), and
the cartridge module (114) comprises the entire liquid fluidic system, the cartridge module (114) comprising a liquid reservoir tank (126, 156, 134), a sample collection device, a drive-less pump mechanism (36'; 122, 154, 128) pumping the liquid from the reservoir tank (126, 156, 134), and the fluidic measuring section (132) for the analyte sensor (150),
wherein, when the cartridge module (114) is in place in the base module (112), the pump mechanism (36'; 122, 154, 128) is connected to the pump drive (16'; 178) and is driven by the pump drive (16'; 178), and the fluidic measuring section (132) is connected to the analyte sensor (150),
the pump mechanism (36'; 122, 154, 128) is configured as a peristaltic membrane pump and is driven pneumatically,
the pump chambers (82) of the membrane pump are covered by a flexible liquid-tight pump membrane (84),
the base module (112) comprises a plurality of independent pump drives (16', 17'; 178) associated to a plurality of independent pump mechanisms (36', 37'; 123, 154, 128) in the cartridge module (114), and
the cartridge module (114) comprises a plastic material plate (190) with groove-like microfluidic channels for connecting the reservoir tank (126, 156, 134), the pump mechanisms (123, 154, 128) and the measuring section (132), the section of the channels being less than 5 mm².

2. The process analysis unit (10; 110) of claim 1, wherein the cartridge module (12; 114) comprises a plurality of reservoir tanks (40, 41; 126, 156, 134).

3. The process analysis unit (10; 110) of one of the preceding claims, wherein the analyte sensor (20; 150) comprises an optical detector (64; 166) allowing the optical determination of an analyte in the fluidic measuring section (60; 132).

4. The process analysis unit (10; 110) of claim 3, wherein the optical detector (64; 166) determines the absorption, the fluorescence or the luminescence of the sample.

5. The process analysis unit (10; 110) of one of the preceding claims, wherein the cartridge module (14; 114) comprises a dialysis membrane (26; 118) for obtaining a sample.

6. The process analysis unit (10; 110) of one of the preceding claims, wherein the analysis unit (110) is designed as an immersion probe.

7. The process analysis unit (10; 110) of one of the preceding claims, wherein the cartridge module (114) is encapsulated in a liquid-tight manner so that the liquids contained therein cannot escape outward.

## Revendications

1. Appareil d'analyse de processus (110) destiné à la détermination d'un analyte dans l'eau, comprenant un module de base (112) et un module de cartouche (114) remplaçable, où
le module de base (112) ne comprend pas de système fluidique liquide, le module de base (112) comprenant un entrainement de pompe (16', 17'; 178) et un capteur d'analyte (150) sans section de mesure fluidique (132), et
le module de cartouche (114) comprend tout le système fluidique liquide, le module de cartouche (114) comprenant un réservoir de liquide (126, 156, 134), un dispositif de collection d'échantillon, un mécanisme de pompe (36'; 112, 154, 128) sans entrainement, pompant le liquide hors du réservoir de liquide (126, 156, 134), et la section de mesure fluidique (132) pour le capteur d'analyte (150), où,
le module de cartouche (114) étant inséré dans le module de base (112), le mécanisme de pompe (36'; 112, 154, 128) est relié avec l'entrainement de pompe (16'; 178) et est entrainé par l'entrainement de pompe (16'; 178), et la section de mesure fluidique (132) est reliée avec le capteur d'analyte (150),
le mécanisme de pompe (36'; 112, 154, 128) est conçu comme pompe à membrane péristaltique et est entrainé pneumatiquement, les chambres de pompage (82) de la pompe à membrane sont recouvertes par une membrane de pompe (84) flexible étanche à liquide,
le module de base (112) comprend plusieurs entrainements de pompe (16', 17'; 178) indépendants l'un de l'autre, associés à plusieurs mécanismes de pompe (36', 37'; 112, 154, 128) indépendants l'un de l'autre dans le module de cartouche (114), et
le module de cartouche (114) comprend une plaque (190) en matière plastique avec des canaux microfluidique en forme de rainures ayant une section transversale du canal inférieur à 5 mm² pour relier le réservoir (126, 156, 134), les mécanismes de pompe (123, 154, 128) et la section de mesure fluidique (132).

2. Appareil d'analyse de processus (10; 110) selon la revendication 1, dans lequel le module de cartouche (12; 114) comprend plusieurs réservoirs (40, 41; 126, 156, 134).

3. Appareil d'analyse de processus (10; 110) selon l'une quelconque des revendications précédentes, dans lequel le capteur d'analyte (20; 150) comprend un détecteur optique (64; 166) permettant la détermination optique d'un analyte dans la section de mesure fluidique (60; 132).

4. Appareil d'analyse de processus (10; 110) selon la revendication 3, dans lequel le détecteur optique (64; 166) détermine l'absorption, la fluorescence ou la luminescence de l'échantillon.

5. Appareil d'analyse de processus (10; 110) selon l'une quelconque des revendications précédentes, dans lequel le module de cartouche (14; 114) comprend une membrane de dialyse (26; 118) pour obtenir un échantillon.

6. Appareil d'analyse de processus (10; 110) selon l'une quelconque des revendications précédentes, dans lequel l'appareil d'analyse (110) est conçu comme une sonde à immersion.

7. Appareil d'analyse de processus (10; 110) selon l'une quelconque des revendications précédentes, dans lequel le module de cartouche (114) est encapsulé de manière étanche à liquide, de sorte que les liquides y contenus ne peuvent pas sortir vers l'extérieur,
